# EUROPEAN PATENT APPLICATION

(11) **EP 4 439 053 A1**
(43) Date of publication of application: **02.10.2024**
(21) Application number: 22895301.4
(22) Date of filing: 14.10.2022
(51) Int. Cl.: G01N 21/65, C12M 1/34, C12M 3/00, G01N 33/483, G06N 3/02, G06N 20/00

(54) **INFORMATION PROCESSING DEVICE, METHOD FOR OPERATING INFORMATION PROCESSING DEVICE, OPERATION PROGRAM FOR INFORMATION PROCESSING DEVICE, METHOD FOR GENERATING CALIBRATED STATE PREDICTION MODEL, AND CALIBRATED STATE PREDICTION MODEL**

(30) Priority: 22.11.2021 JP 2021189571
(71) Applicant: FUJIFILM Corporation, Tokyo 106-8620 (JP)
(72) Inventor: SUGITA, Yui, Ashigarakami-gun, Kanagawa 258-8577 (JP)
(74) Representative: HGF
(86) International application number: PCT/JP2022/038480
(87) International publication number: WO 2023/090015

(57) **Abstract**

An information processing apparatus is an information processing apparatus that predicts a state of a component in a suspension in which biological molecules are dispersed as the components in a liquid, based on spectrum measurement data obtained by measuring a spectrum of electromagnetic waves emitted from the suspension, the information processing apparatus including: a processor, in which the processor uses a calibrated state predictive model calibrated by using at least two types of calibration data of first calibration data and second calibration data, the first calibration data including first spectrum measurement data, which is the spectrum measurement data obtained from a first suspension containing a first component, and first component relation information related to the first component as explanatory variables, and including first state relation information related to a state of the first component as a response variable, and the second calibration data including second spectrum measurement data, which is the spectrum measurement data obtained from a second suspension containing a second component, and second component relation information related to the second component as explanatory variables, and including second state relation information related to a state of the second component as a response variable, acquires target component relation information related to a target component, which is a target of which the state is predicted, and target spectrum measurement data, which is the spectrum measurement data obtained from a target suspension containing the target component, and applies the target component relation information and the target spectrum measurement data to the calibrated state predictive model, and causes the calibrated state predictive model to output a target state prediction result obtained by predicting the state of the target component in the target suspension.

## Description

### BACKGROUND OF THE INVENTION

### 1. Field of the Invention

The technology of the present disclosure relates to an information processing apparatus, an operation method of an information processing apparatus, an operation program of an information processing apparatus, a generation method of a calibrated state predictive model, and a calibrated state predictive model.

### 2. Description of the Related Art

For example, a manufacturing process of a bio-pharmaceutical containing a biological molecule such as a protein, such as a monoclonal antibody, as an active ingredient is known. In such a manufacturing process, a suspension in which various components including the active ingredient are dispersed in a liquid is often produced. It is important to monitor a state of the target component (for example, the protein or an impurity derived from the protein) in the suspension in a manufacturing line in order to successfully lead the ongoing manufacturing process.

JP2016-128822A describes a technology of predicting a concentration as a state of a target component in a manufacturing line. Specifically, in JP2016-128822A, a Raman spectrum of the suspension is measured in the manufacturing line, and the concentration of the target component is predicted from the Raman spectrum by using a linear model.

### SUMMARY OF THE INVENTION

The linear model described in JP2016-128822A is, for example, a dedicated model specialized in one target component A. Therefore, in a case in which a concentration of a target component B different from the target component A is predicted, it is necessary to newly generate a dedicated model for predicting the concentration of the target component B. In the manufacture of various bio-pharmaceuticals containing different antibodies as the active ingredients, it is necessary to generate a dedicated model for each target component, which is extremely inefficient.

One embodiment according to the technology of the present disclosure provides an information processing apparatus, an operation method of an information processing apparatus, an operation program of an information processing apparatus, a generation method of a calibrated state predictive model, and a calibrated state predictive model, which can efficiently predict a state of a target component in a suspension in which biological molecules are dispersed in a liquid as components.

The present disclosure relates to an information processing apparatus that predicts a state of a component in a suspension in which biological molecules are dispersed as the components in a liquid, based on spectrum measurement data obtained by measuring a spectrum of electromagnetic waves emitted from the suspension, the information processing apparatus comprising: a processor, in which the processor uses a calibrated state predictive model calibrated by using at least two types of calibration data of first calibration data and second calibration data, the first calibration data including first spectrum measurement data, which is the spectrum measurement data obtained from a first suspension containing a first component, and first component relation information related to the first component as explanatory variables, and including first state relation information related to a state of the first component as a response variable, and the second calibration data including second spectrum measurement data, which is the spectrum measurement data obtained from a second suspension containing a second component, and second component relation information related to the second component as explanatory variables, and including second state relation information related to a state of the second component as a response variable, acquires target component relation information related to a target component, which is a target of which the state is predicted, and target spectrum measurement data, which is the spectrum measurement data obtained from a target suspension containing the target component, and applies the target component relation information and the target spectrum measurement data to the calibrated state predictive model, and causes the calibrated state predictive model to output a target state prediction result obtained by predicting the state of the target component in the target suspension.

It is preferable that the calibrated state predictive model includes a first model that outputs a temporary prediction result of the state of the target component in accordance with the target spectrum measurement data, and a second model that outputs the target state prediction result in accordance with the target component relation information and the temporary prediction result.

It is preferable that the target component is different from a component used to obtain the calibration data.

It is preferable that the processor performs preprocessing for at least any one of noise removal, peak separation, or peak emphasis on the target spectrum measurement data, and then applies the preprocessed target spectrum measurement data to the calibrated state predictive model.

It is preferable that the first component, the second component, and the target component are proteins.

It is preferable that the first component relation information, the second component relation information, and the target component relation information include information on a compositional ratio of an amino acid in the protein.

It is preferable that the protein is an antibody.

It is preferable that the first component relation information, the second component relation information, and the target component relation information include information on a subclass of the antibody.

It is preferable that the spectrum is a Raman spectrum.

It is preferable that the calibrated state predictive model is a machine learning model trained using the calibration data as training data.

It is preferable that the state is a concentration, the first state relation information is a measurement value of a concentration of the first component, the second state relation information is a measurement value of a concentration of the second component, and the target state prediction result is a prediction value of a concentration of the target component.

The present disclosure relates to an operation method of an information processing apparatus that predicts a state of a component in a suspension in which biological molecules are dispersed as the components in a liquid, based on spectrum measurement data obtained by measuring a spectrum of electromagnetic waves emitted from the suspension, the operation method comprising: using a calibrated state predictive model calibrated by using at least two types of calibration data of first calibration data and second calibration data, the first calibration data including first spectrum measurement data, which is the spectrum measurement data obtained from a first suspension containing a first component, and first component relation information related to the first component as explanatory variables, and including first state relation information related to a state of the first component as a response variable, and the second calibration data including second spectrum measurement data, which is the spectrum measurement data obtained from a second suspension containing a second component, and second component relation information related to the second component as explanatory variables, and including second state relation information related to a state of the second component as a response variable; acquiring target component relation information related to a target component, which is a target of which the state is predicted, and target spectrum measurement data, which is the spectrum measurement data obtained from a target suspension containing the target component; and applying the target component relation information and the target spectrum measurement data to the calibrated state predictive model, and causing the calibrated state predictive model to output a target state prediction result obtained by predicting the state of the target component in the target suspension.

The present disclosure relates to an operation program of an information processing apparatus that predicts a state of a component in a suspension in which biological molecules are dispersed as the components in a liquid, based on spectrum measurement data obtained by measuring a spectrum of electromagnetic waves emitted from the suspension, the operation program causing a computer to execute a process comprising: using a calibrated state predictive model calibrated by using at least two types of calibration data of first calibration data and second calibration data, the first calibration data including first spectrum measurement data, which is the spectrum measurement data obtained from a first suspension containing a first component, and first component relation information related to the first component as explanatory variables, and including first state relation information related to a state of the first component as a response variable, and the second calibration data including second spectrum measurement data, which is the spectrum measurement data obtained from a second suspension containing a second component, and second component relation information related to the second component as explanatory variables, and including second state relation information related to a state of the second component as a response variable; acquiring target component relation information related to a target component, which is a target of which the state is predicted, and target spectrum measurement data, which is the spectrum measurement data obtained from a target suspension containing the target component; and applying the target component relation information and the target spectrum measurement data to the calibrated state predictive model, and causing the calibrated state predictive model to output a target state prediction result obtained by predicting the state of the target component in the target suspension.

The present disclosure relates to a generation method of a calibrated state predictive model that predicts a state of a component in a suspension in which biological molecules are dispersed as the components in a liquid, based on spectrum measurement data obtained by measuring a spectrum of electromagnetic waves emitted from the suspension, the generation method comprising: acquiring at least two types of calibration data of first calibration data and second calibration data, the first calibration data including first spectrum measurement data, which is the spectrum measurement data obtained from a first suspension containing a first component, and first component relation information related to the first component as explanatory variables, and including first state relation information related to a state of the first component as a response variable, and the second calibration data including second spectrum measurement data, which is the spectrum measurement data obtained from a second suspension containing a second component, and second component relation information related to the second component as explanatory variables, and including second state relation information related to a state of the second component as a response variable; and generating the calibrated state predictive model by using the calibration data.

It is preferable that the generation method of a calibrated state predictive model further comprises: inputting the explanatory variables of the calibration data to a machine learning model as input data for training, and causing the machine learning model to output a state prediction result for training obtained by predicting the state; and updating the machine learning model based on a result of comparison between the state prediction result for training and the response variable of the calibration data, in which the machine learning model is made to be the calibrated state predictive model by repeatedly performing inputting the explanatory variables to the machine learning model, causing the machine learning model to output the state prediction result for training, and updating the machine learning model, while changing the calibration data.

The present disclosure relates to a calibrated state predictive model that predicts a state of a component in a suspension in which biological molecules are dispersed as the components in a liquid, based on spectrum measurement data obtained by measuring a spectrum of electromagnetic waves emitted from the suspension, in which the calibrated state predictive model is generated by using at least two types of calibration data of first calibration data and second calibration data, the first calibration data including first spectrum measurement data, which is the spectrum measurement data obtained from a first suspension containing a first component, and first component relation information related to the first component as explanatory variables, and including first state relation information related to a state of the first component as a response variable, and the second calibration data including second spectrum measurement data, which is the spectrum measurement data obtained from a second suspension containing a second component, and second component relation information related to the second component as explanatory variables, and including second state relation information related to a state of the second component as a response variable, and causes a computer to execute a function of, in a case in which target component relation information related to a target component, which is a target of which the state is predicted, and target spectrum measurement data, which is the spectrum measurement data obtained from a target suspension containing the target component, are applied, outputting a target state prediction result obtained by predicting the state of the target component in the target suspension.

The present disclosure relates to an information processing apparatus that stores the calibrated state predictive model described above.

According to the technology of the present disclosure, it is possible to provide the information processing apparatus, the operation method of the information processing apparatus, the operation program of the information processing apparatus, the generation method of the calibrated state predictive model, and the calibrated state predictive model, which can efficiently predict the state of the target component in the suspension in which the biological molecules are dispersed in the liquid as the components.

### BRIEF DESCRIPTION OF THE DRAWINGS

Fig. 1 is a diagram showing an outline of a manufacturing process of a bio-pharmaceutical.
Fig. 2 is a diagram showing a state in which target spectrum measurement data obtained by measuring a Raman spectrum of a target first purified liquid with a Raman spectrometer, and target component relation information of a target antibody in the target first purified liquid are acquired by an information processing apparatus.
Fig. 3 is a diagram showing the target spectrum measurement data.
Fig. 4 is a diagram showing the target component relation information.
Fig. 5 is a block diagram of a computer constituting the information processing apparatus.
Fig. 6 is a block diagram of a CPU of the computer constituting the information processing apparatus.
Fig. 7 is a diagram showing an outline of processing performed by a preprocessing unit.
Fig. 8 is a diagram showing sparse processing as peak emphasis processing.
Fig. 9 is a diagram showing an outline of processing performed by a prediction unit.
Fig. 10 is a diagram showing a neural network constituting a first model and a second model.
Fig. 11 is a diagram showing a structure of a training data group.
Fig. 12 is a diagram showing the training data group.
Fig. 13 is a diagram showing a state in which preprocessing is performed on the spectrum measurement data of the training data to obtain the preprocessed spectrum measurement data.
Fig. 14 is a diagram showing an outline of processing in a training phase of the first model.
Fig. 15 is a diagram showing an outline of processing in a training phase of the second model.
Fig. 16 is a flowchart showing a procedure of acquiring training data and storing the acquired training data in a storage.
Fig. 17 is a flowchart showing a processing procedure in the training phase of the first model.
Fig. 18 is a flowchart showing a processing procedure in the training phase of the second model.
Fig. 19 is a flowchart showing a processing procedure of the information processing apparatus.

### DESCRIPTION OF THE PREFERRED EMBODIMENTS

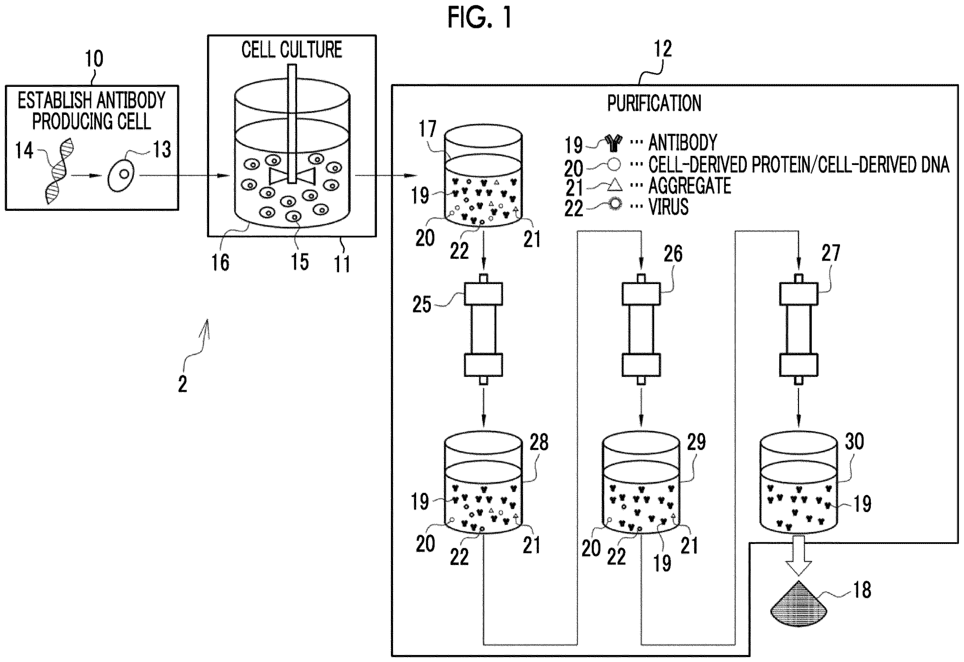

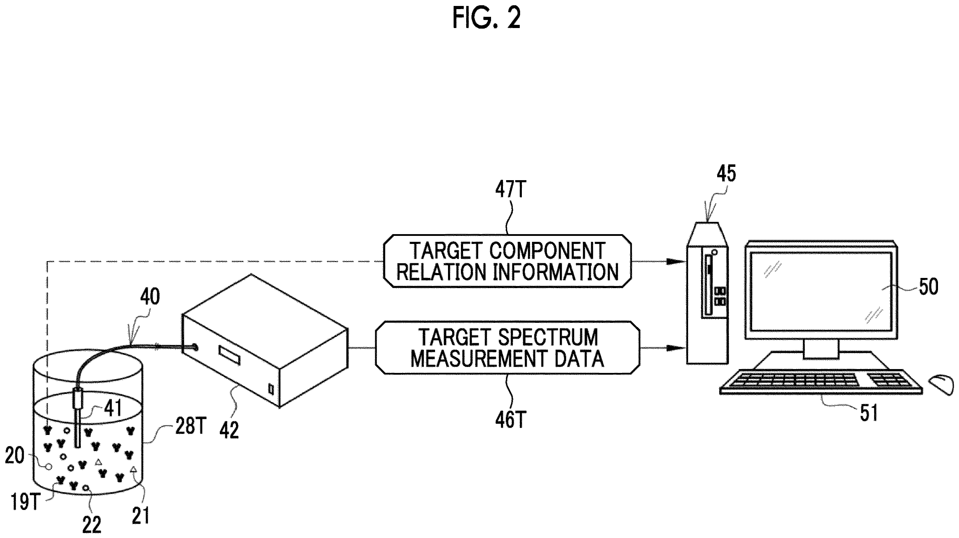

As shown in Fig. 1 as an example, a manufacturing process 2 of a bio-pharmaceutical to which an information processing apparatus 45 (see Fig. 2) according to the technology of the present disclosure is applied is roughly divided into a first process 10, a second process 11, and a third process 12. The first process 10 is a process of incorporating an antibody gene 14 into a host cell 13 such as Chinese hamster ovary (CHO) cells to establish an antibody producing cell 15. The second process is a process of cell culture of the antibody producing cell 15 in a culture tank 16.

The third process 12 is a process of purifying a drug substance 18 of the bio-pharmaceutical from a culture supernatant liquid 17. The culture supernatant liquid 17 is a solution obtained by removing cells from a culture liquid in the culture tank 16 after the second process 11. The immunoglobulins produced by the antibody producing cell 15, that is, antibodies 19 are dispersed in the culture supernatant liquid 17. The antibody 19 is, for example, a monoclonal antibody, and is an active component of the bio-pharmaceutical. In addition, in the culture supernatant liquid 17, impurities such as a cell-derived protein/cell-derived deoxyribonucleic acid (DNA) 20 and an aggregate 21 of the antibody 19, or a virus 22 are also dispersed, in addition to the antibody 19. The antibody 19 is an example of a "biological molecule" and a "protein" according to the technology of the present disclosure. It should be noted that the "biological molecule" means a substance obtained from a cell, a cellular organelle, a cellular molecule, a gene recombinant, a natural non-synthesized chemical substance-derived organism, or the like.

An immunoaffinity chromatography device 25, a cation chromatography device 26, and an anion chromatography device 27 are used in the third process 12. The culture supernatant liquid 17 is introduced into the immunoaffinity chromatography device 25. The immunoaffinity chromatography device 25 extracts the antibody 19 from the culture supernatant liquid 17 by using a column in which a ligand such as a protein A having an affinity for the antibody 19 is immobilized on a carrier, thereby generating a first purified liquid 28. Although not shown, the first purified liquid 28 is subjected to a treatment for inactivating the virus 22 (hereinafter, referred to as a virus inactivation treatment).

The first purified liquid 28 after the virus inactivation treatment is introduced into the cation chromatography device 26. The cation chromatography device 26 extracts the antibody 19 from the first purified liquid 28 by using a column having a cation exchanger as a stationary phase, to generate a second purified liquid 29. The second purified liquid 29 is introduced into the anion chromatography device 27. The anion chromatography device 27 extracts the antibody 19 from the second purified liquid 29 by using a column having an anion exchanger as a stationary phase, to generate a third purified liquid 30. Although not shown, a treatment of removing the virus is performed on the third purified liquid 30. Thereafter, the third purified liquid 30 is subjected to a concentration/filtration treatment by an ultrafiltration (UF) and a diafiltration (DF), whereby the drug substance 18 is purified. By sequentially performing a component separation treatment using such a plurality of types of the chromatography devices 25 to 27, the impurities and the virus 22 are gradually removed, and a purity of the antibody 19 is gradually increased.

Hereinafter, for the sake of simplicity, as an example, a case will be described in which the concentration of the antibody 19 in the first purified liquid 28 is predicted. Hereinafter, the antibody 19 of which the concentration is predicted will be referred to as a target antibody 19T, and the first purified liquid 28 containing the target antibody 19T will be referred to as a target first purified liquid 28T. The target antibody 19T is an example of a "target component" according to the technology of the present disclosure. The target first purified liquid 28T is an example of a "target suspension" according to the technology of the present disclosure. The concentration is an example of a "state" according to the technology of the present disclosure. It should be noted that the "state" is an indicator representing physicochemical features of the target component.

As shown in Fig. 2 as an example, in the present embodiment, a Raman spectrum of the target first purified liquid 28T is measured in the third process 12 by using a Raman spectrometer 40. The Raman spectrometer 40 is a device that evaluates a substance by using characteristics of Raman scattered light. In a case in which the substance is irradiated with excitation light, the Raman scattered light having a wavelength different from the excitation light is generated by an interaction between the excitation light and the substance. A wavelength difference between the excitation light and the Raman scattered light corresponds to an energy distribution of molecular vibration possessed by the substance. Therefore, the Raman scattered light having different wave numbers can be obtained between the substances having different molecular structures. Out of a Stokes ray and an anti-Stokes ray, the Stokes ray is preferably used as the Raman scattered light. The Raman spectrum is an example of a "spectrum of electromagnetic waves" according to the technology of the present disclosure.

The Raman spectrometer 40 is configured by a probe 41 and an analyzer 42. A distal end of the probe 41 is immersed in the target first purified liquid 28T. The probe 41 emits the excitation light from an emission port at the distal end and receives the Raman scattered light generated by the interaction between the excitation light and the target first purified liquid 28T, by a light-receiving unit disposed at the distal end. The probe 41 outputs the received Raman scattered light to the analyzer 42. It should be noted that, in the present embodiment, laser light is used as the excitation light, the output of the laser light is set to 200 mW, the central wavelength is set to 785 nm, and the irradiation time is set to 1 second. It should be noted that the Raman spectrometer 40 is not limited to the type in which the probe 41 having the light-receiving unit is immersed in the liquid and used, and may be a type in which a flow cell having a light-receiving unit is installed in a flow channel and used.

The analyzer 42 generates spectrum measurement data by decomposing the Raman scattered light for each wave number and deriving the intensity of the Raman scattered light for each wave number. The analyzer 42 is connected to the information processing apparatus 45 in a mutually communicable manner through a computer network such as a local area network (LAN). The analyzer 42 transmits the generated spectrum measurement data to the information processing apparatus 45 as target spectrum measurement data 46T. The information processing apparatus 45 receives the target spectrum measurement data 46T from the analyzer 42. The information processing apparatus 45 accepts target component relation information 47T, which is information related to the target antibody 19T. Here, the "component relation information" means information that is unique to the component regardless of the state of the component. The "component relation information" is information that affects the spectrum such as the Raman spectrum.

The information processing apparatus 45 is, for example, a desktop personal computer, and comprises a display 50 on which various screens provided with a graphical user interface (GUI) are displayed, and an input device 51 such as a keyboard and a mouse for performing an operation through the GUI. The target component relation information 47T is input by, for example, an operator of the information processing apparatus 45 via the input device 51. It should be noted that the information processing apparatus 45 may be a laptop personal computer or a tablet terminal.

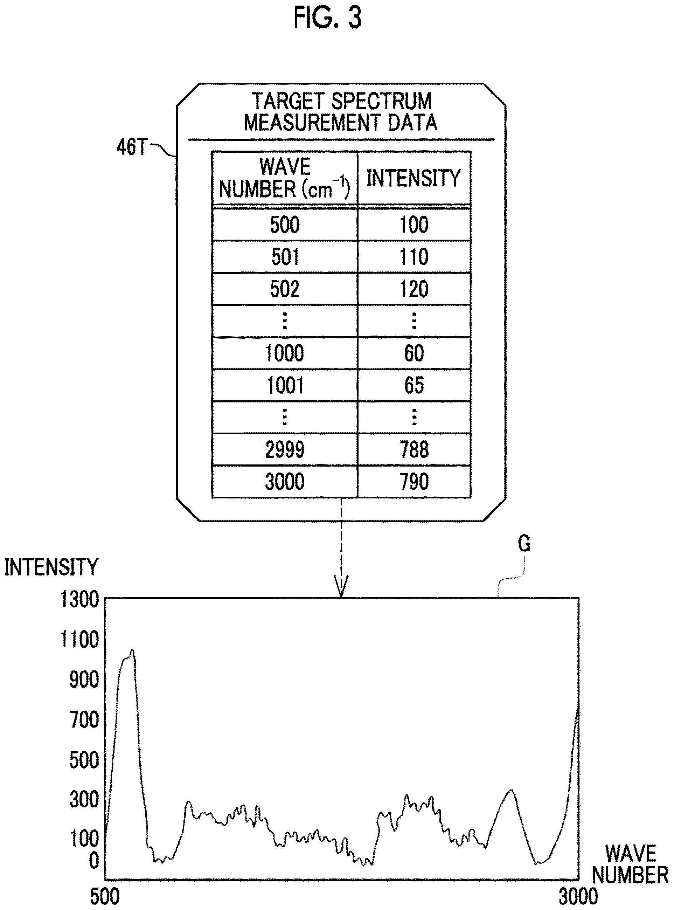

As shown in Fig. 3 as an example, the target spectrum measurement data 46T is data in which the intensity of the Raman scattered light for each wave number is registered. In Fig. 3, the target spectrum measurement data 46T is data in which the intensity of the scattered light in a wave number range of 500 cm⁻¹ to 3000 cm⁻¹ is derived in an interval of 1 cm⁻¹. It should be noted that a graph G shown in a lower part of Fig. 3 is a graph in which the intensities of the target spectrum measurement data 46T are plotted for each wave number and connected by a line.

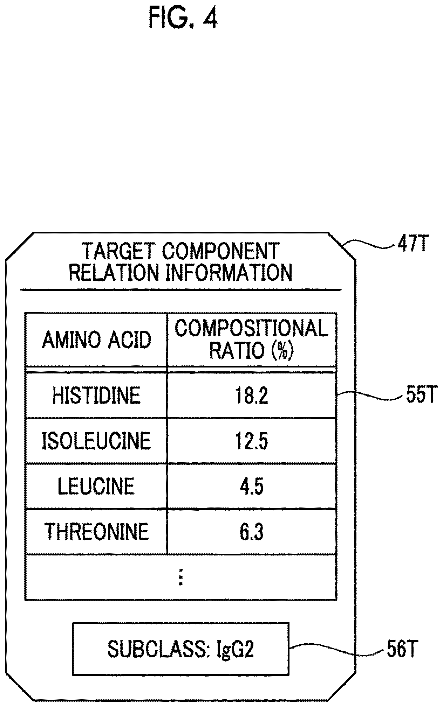

As shown in Fig. 4 as an example, the target component relation information 47T includes information (hereinafter, referred to as target amino acid compositional ratio information) 55T on a compositional ratio of an amino acid in the target antibody 19T and information (hereinafter, referred to as target subclass information) 56T on a subclass of the target antibody 19T. The target amino acid compositional ratio information 55T is information in which the compositional ratio (%) of various amino acids, such as histidine and leucine, is registered. The target subclass information 56T is information in which the subclasses of the target antibody 19T, such as immunoglobulin (Ig) G1, IgG2, IgG3, and IgG4, are registered. Fig. 4 shows a case in which IgG2 is registered as the subclass. The target amino acid compositional ratio information 55T may be information on the compositional ratio of any amino acid of the heavy chain or the light chain in the target antibody 19T, but is more preferably information on the compositional ratio of both the amino acids of the heavy chain and the light chain.

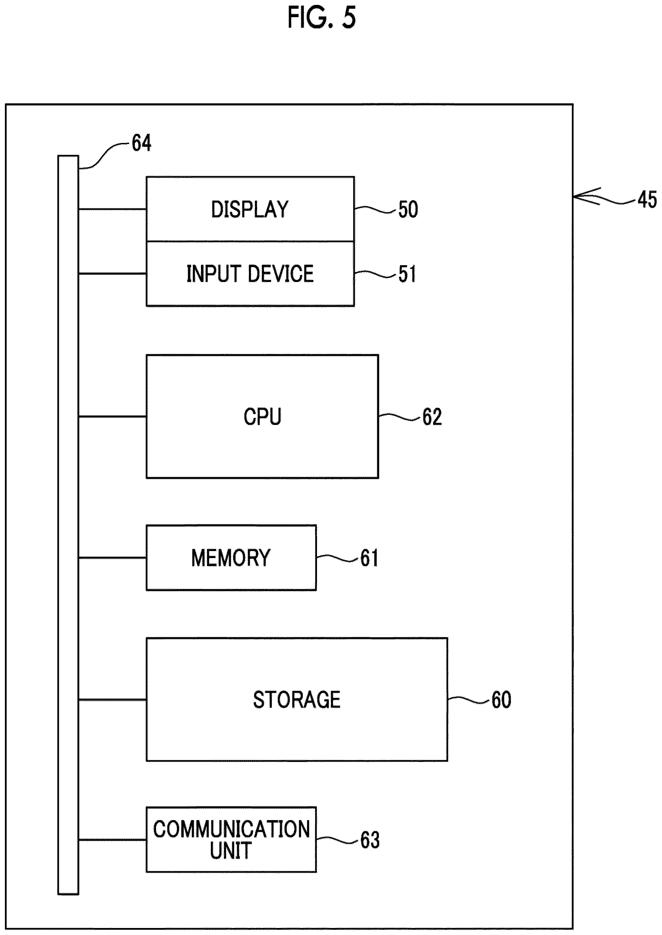

For example, as shown in Fig. 5, the computer constituting the information processing apparatus 45 comprises a storage 60, a memory 61, a central processing unit (CPU) 62, and a communication unit 63, in addition to the display 50 and the input device 51 described above. These units are connected to each other via a busline 64.

The storage 60 is a hard disk drive that is incorporated in the computer constituting the information processing apparatus 45 or connected to the computer through a cable or a network. Alternatively, the storage 60 is a disk array in which a plurality of hard disk drives are mounted. The storage 60 stores a control program such as an operating system, various application programs, various data associated with these programs, and the like. It should be noted that a solid state drive may be used instead of the hard disk drive.

The memory 61 is a work memory for the CPU 62 to execute processing. The CPU 62 loads the program stored in the storage 60 into the memory 61, and executes processing in accordance with the program. Accordingly, the CPU 62 integrally controls the respective units of the computer. The CPU 62 is an example of a "processor" according to the technology of the present disclosure. The communication unit 63 performs transmission control of various information with an external device, such as the Raman spectrometer 40. It should be noted that the memory 61 may be incorporated in the CPU 62.

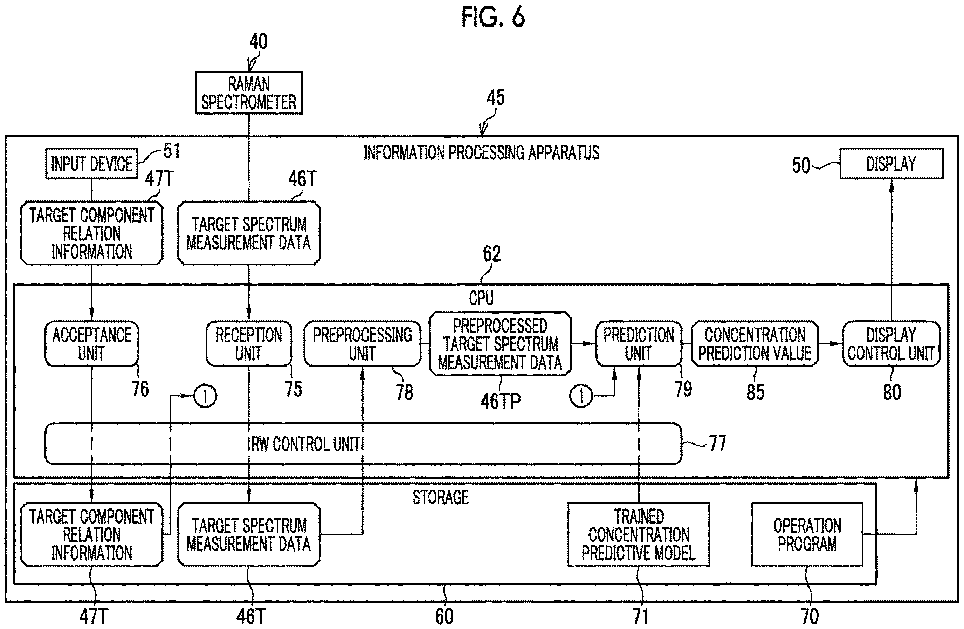

As shown in Fig. 6, for example, an operation program 70 is stored in the storage 60 of the information processing apparatus 45. The operation program 70 is an application program for causing the computer to function as the information processing apparatus 45. In other words, the operation program 70 is an example of an "operation program of an information processing apparatus" according to the technology of the present disclosure. The storage 60 also stores a trained concentration predictive model 71. The trained concentration predictive model 71 is an example of a "calibrated state predictive model" according to the technology of the present disclosure. The storage 60 stores data of various screens to be displayed on the display 50, and the like.

In a case in which the operation program 70 is activated, the CPU 62 of the computer constituting the information processing apparatus 45 functions as a reception unit 75, an acceptance unit 76, a read/write (hereinafter, abbreviated as RW) control unit 77, a preprocessing unit 78, a prediction unit 79, and a display control unit 80 in cooperation with the memory 61 and the like.

The reception unit 75 receives the target spectrum measurement data 46T from the Raman spectrometer 40. In this way, by receiving the target spectrum measurement data 46T via the reception unit 75, the CPU 62 acquires the target spectrum measurement data 46T. The reception unit 75 outputs the target spectrum measurement data 46T to the RW control unit 77.

The acceptance unit 76 accepts the target component relation information 47T input by the operator via the input device 51. In this way, the CPU 62 acquires the target component relation information 47T by accepting the target component relation information 47T via the acceptance unit 76. The acceptance unit 76 outputs the target component relation information 47T to the RW control unit 77.

The RW control unit 77 controls the readout of various data stored in the storage 60 and the storage of various data in the storage 60. For example, the RW control unit 77 stores the target spectrum measurement data 46T from the reception unit 75 and the target component relation information 47T from the acceptance unit 76, in the storage 60. The RW control unit 77 reads out the target spectrum measurement data 46T from the storage 60, and outputs the read out target spectrum measurement data 46T to the preprocessing unit 78. The RW control unit 77 reads out the target component relation information 47T from the storage 60, and outputs the read out target component relation information 47T to the prediction unit 79. Further, the RW control unit 77 reads out the trained concentration predictive model 71 from the storage 60, and outputs the read out trained concentration predictive model 71 to the prediction unit 79.

The preprocessing unit 78 performs the preprocessing on the target spectrum measurement data 46T to make the target spectrum measurement data 46T as preprocessed target spectrum measurement data 46TP. The preprocessing unit 78 outputs the preprocessed target spectrum measurement data 46TP to the prediction unit 79.

The prediction unit 79 applies the preprocessed target spectrum measurement data 46TP and the target component relation information 47T to the trained concentration predictive model 71, and causes the trained concentration predictive model 71 to output a concentration prediction value 85 obtained by predicting the concentration of the target antibody 19T. The prediction unit 79 outputs the concentration prediction value 85 to the display control unit 80. The concentration prediction value 85 is an example of a "target state prediction result" according to the technology of the present disclosure.

The display control unit 80 controls display of various screens on the display 50. For example, the display control unit 80 displays an input screen of the target component relation information 47T on the display 50. The display control unit 80 causes the display 50 to display a notification screen for notifying the operator of the concentration prediction value 85 from the prediction unit 79.

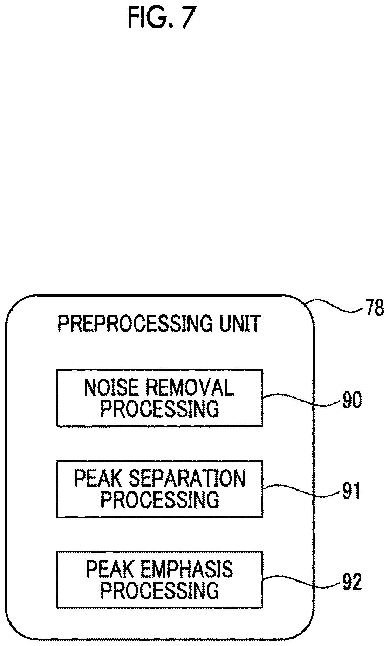

As shown in Fig. 7 as an example, the preprocessing unit 78 performs noise removal processing 90, peak separation processing 91, and peak emphasis processing 92 on the target spectrum measurement data 46T, as the preprocessing. Examples of the noise removal processing 90 include smoothing processing by a Savitzky-Golay (SG) method and baseline correction processing. The peak separation processing 91 is differential processing (also referred to as derivative calculation processing) or the like. The peak emphasis processing 92 is normalization processing (also referred to as standardization processing), averaging processing, for example, dimension reduction processing by principal component analysis, sparse processing, and the like.

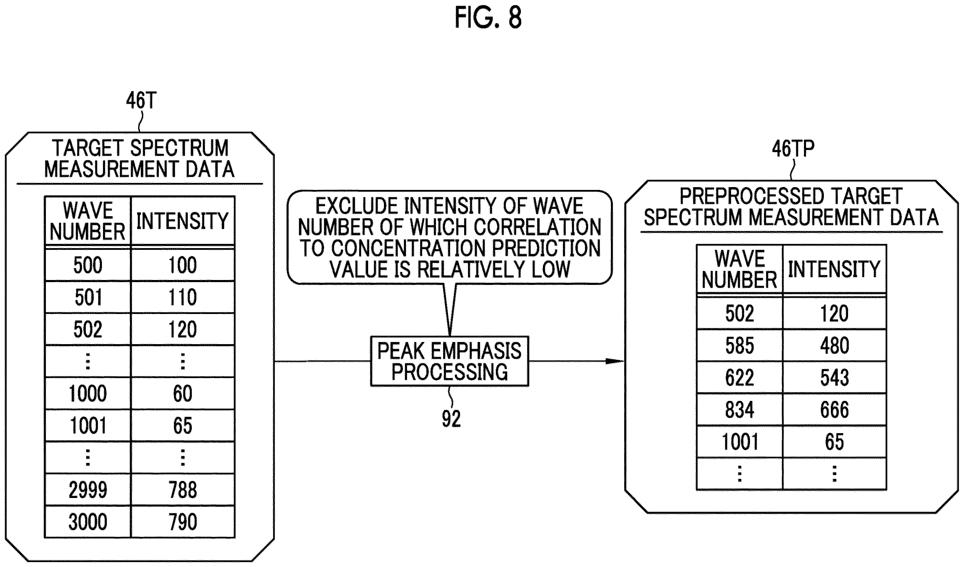

As shown in Fig. 8 as an example, the sparse processing as the peak emphasis processing 92 is processing of excluding the intensity of the wave number of which the correlation to the concentration prediction value 85 is relatively low among the intensities of the respective wave numbers of the target spectrum measurement data 46T. Through this sparse processing, the number of intensities, that is, the number of data of the preprocessed target spectrum measurement data 46TP is significantly smaller than the number of data (2501 in the present example) of the target spectrum measurement data 46T. The number of data of the preprocessed target spectrum measurement data 46TP is, for example, preferably 5 or more and less than 1000, more preferably 5 or more and less than 800, and still more preferably 5 or more and less than 500.

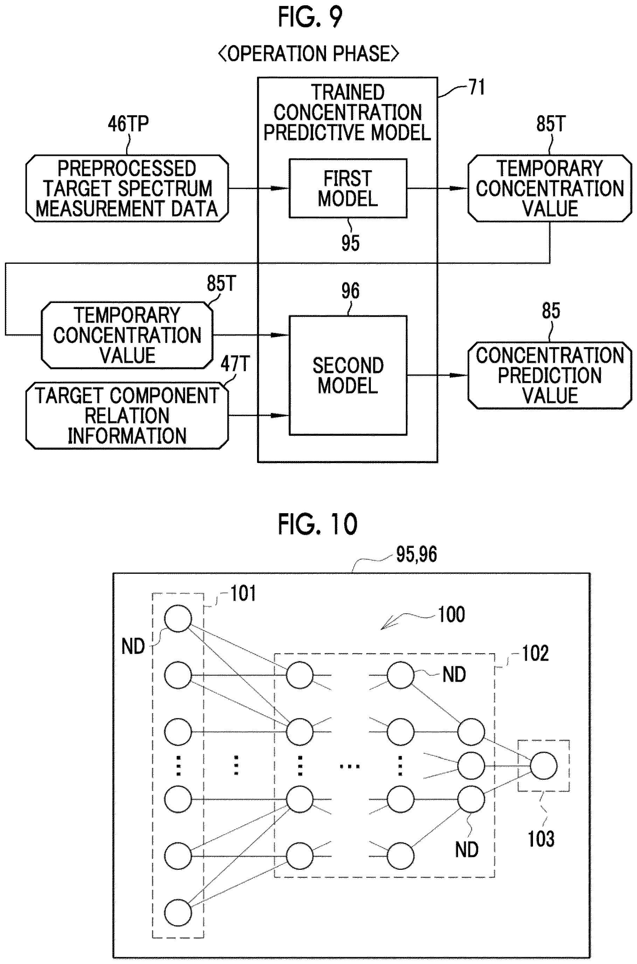

As shown in Fig. 9 as an example, the trained concentration predictive model 71 comprises a first model 95 and a second model 96. The prediction unit 79 inputs the preprocessed target spectrum measurement data 46TP to the first model 95, and causes the first model 95 to output a temporary concentration prediction value 85T, which is a temporary prediction value of the concentration of the target antibody 19T. That is, the first model 95 is a model that outputs the temporary concentration prediction value 85T in accordance with the preprocessed target spectrum measurement data 46TP. The temporary concentration prediction value 85T is an example of a "temporary prediction result" according to the technology of the present disclosure.

Then, the prediction unit 79 inputs the temporary concentration prediction value 85T output by the first model 95 and the target component relation information 47T to the second model 96, and causes the second model 96 to output the concentration prediction value 85. That is, the second model 96 is a model that outputs the concentration prediction value 85 in accordance with the target component relation information 47T and the temporary concentration prediction value 85T. In this way, the prediction unit 79 predicts the concentration prediction value 85 in two stages by using the first model 95 and the second model 96.

As shown in Fig. 10 as an example, the first model 95 and the second model 96 are constructed by a neural network 100. As is well known, the neural network 100 includes an input layer 101, a hidden layer (also referred to as an intermediate layer) 102, and an output layer 103. Each of the input layer 101, the hidden layer 102, and the output layer 103 includes a plurality of nodes ND. A coefficient indicating the strength of the connection between the nodes ND is set between the node ND of the input layer 101 and the node ND of the hidden layer 102, between the nodes ND in the hidden layer 102, and between the node ND of the hidden layer 102 and the node ND of the output layer 103. A suitable activation function, such as a linear function or a rectified linear unit (ReLU) function, is set for the node ND of the output layer 103.

The intensity of each wave number of the preprocessed target spectrum measurement data 46TP is input to each node ND of the input layer 101 of the first model 95. The temporary concentration prediction value 85T is output from the node ND of the output layer 103 of the first model 95. On the other hand, the temporary concentration prediction value 85T, and each compositional ratio of the target amino acid compositional ratio information 55T and the target subclass information 56T in the target component relation information 47T are input to each node ND of the input layer 101 of the second model 96. The concentration prediction value 85 is output from the node ND of the output layer 103 of the second model 96.

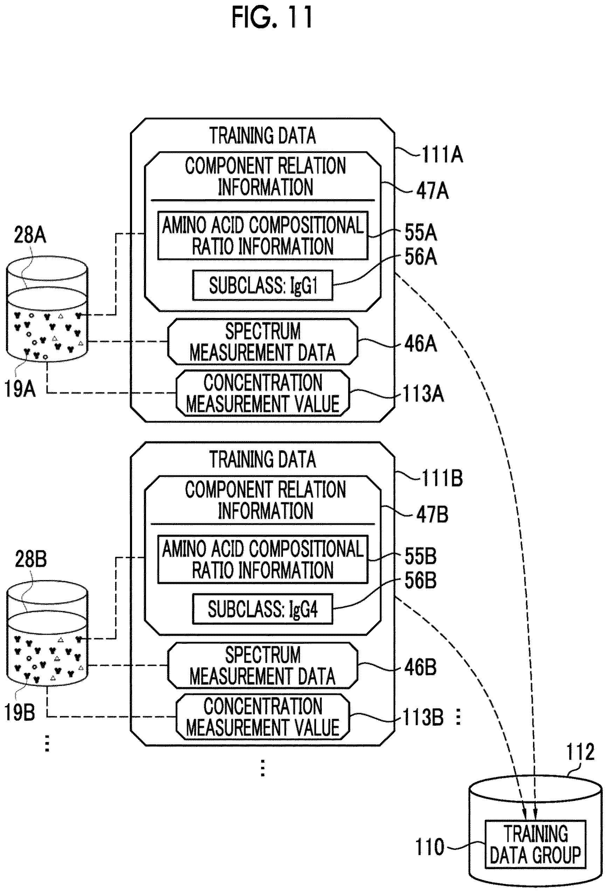

As shown in Fig. 11 as an example, a training data group 110 for generating the trained concentration predictive model 71 is a set of a plurality of training data 111A, 111B, ... (hereinafter, may be collectively referred to as training data 111). The training data group 110 is stored in a storage 112 of a learning apparatus in which the machine learning model is the trained concentration predictive model 71 by, for example, training the machine learning model using the training data 111. It should be noted that the learning apparatus may be an apparatus different from the information processing apparatus 45, or may be the information processing apparatus 45. In a case in which the information processing apparatus 45 has the functions of the learning apparatus, the storage 112 is the storage 60 of the information processing apparatus 45. The training data 111 is an example of "calibration data" according to the technology of the present disclosure.

The training data 111 can be collected from a commercially available bio-pharmaceutical, a bio-pharmaceutical actually manufactured by a small-scale equipment, or the like. The training data 111 can also be acquired in, for example, the manufacturing process 2 of the bio-pharmaceutical of the past. The training data 111 is composed of a set of spectrum measurement data 46, component relation information 47, and a concentration measurement value 113. The spectrum measurement data 46 is data obtained by measuring the Raman spectrum of the first purified liquid 28, which is purified in the manufacturing process 2 of the bio-pharmaceutical of the past, via the Raman spectrometer 40, in the same manner as in a case of the target spectrum measurement data 46T shown in Fig. 2. The component relation information 47 is information related to the antibody 19 in the manufacturing process 2 of the bio-pharmaceutical of the past. The component relation information 47 includes amino acid compositional ratio information 55 and subclass information 56 of the antibody 19 in the manufacturing process 2 of the bio-pharmaceutical of the past. The concentration measurement value 113 is a value obtained by actually measuring the concentration of the antibody 19 in the first purified liquid 28 purified in the manufacturing process 2 of the bio-pharmaceutical of the past, for example, by using a method such as high performance liquid chromatography (HPLC).

In Fig. 11, two training data 111A and 111B are shown as the training data 111. The training data 111A is data acquired from an antibody 19A and a first purified liquid 28A containing the antibody 19A, and the training data 111B is data acquired from an antibody 19B and a first purified liquid 28B containing the antibody 19B. Specifically, the training data 111A is composed of a set of spectrum measurement data 46A obtained by measuring the Raman spectrum of the first purified liquid 28A via the Raman spectrometer 40, component relation information 47A related to the antibody 19A, and a concentration measurement value 113A of the antibody 19A in the first purified liquid 28A. The training data 111B is composed of a set of spectrum measurement data 46B obtained by measuring the Raman spectrum of the first purified liquid 28B via the Raman spectrometer 40, component relation information 47B related to the antibody 19B, and a concentration measurement value 113B of the antibody 19B in the first purified liquid 28B.

In accordance with subclass information 56A, the subclass of the antibody 19A is IgG1. On the other hand, in accordance with subclass information 56B, the subclass of the antibody 19B is IgG4. That is, the subclass of the antibody 19 is different between the training data 111A and the training data 111B. Therefore, the types of the training data 111A and the training data 111B are different from each other. In accordance with the target subclass information 56T shown in Fig. 4, the subclass of the target antibody 19T is IgG2. The target antibody 19T is different from antibodies 19Aand 19B.

The antibody 19Ais an example of a "first component" according to the technology of the present disclosure, and the first purified liquid 28A is an example of a "first suspension" according to the technology of the present disclosure. The training data 111A is an example of "first calibration data" according to the technology of the present disclosure. The spectrum measurement data 46A is an example of "first spectrum measurement data" according to the technology of the present disclosure, and the component relation information 47A is an example of "first component relation information" according to the technology of the present disclosure. Further, the concentration measurement value 113A is an example of "first state relation information" according to the technology of the present disclosure. The antibody 19B is an example of a "second component" according to the technology of the present disclosure, and the second purified liquid 28B is an example of a "second suspension" according to the technology of the present disclosure. The training data 111B is an example of "second calibration data" according to the technology of the present disclosure. The spectrum measurement data 46B is an example of "second spectrum measurement data" according to the technology of the present disclosure, and the component relation information 47B is an example of "second component relation information" according to the technology of the present disclosure. Further, the concentration measurement value 113B is an example of "second state relation information" according to the technology of the present disclosure.

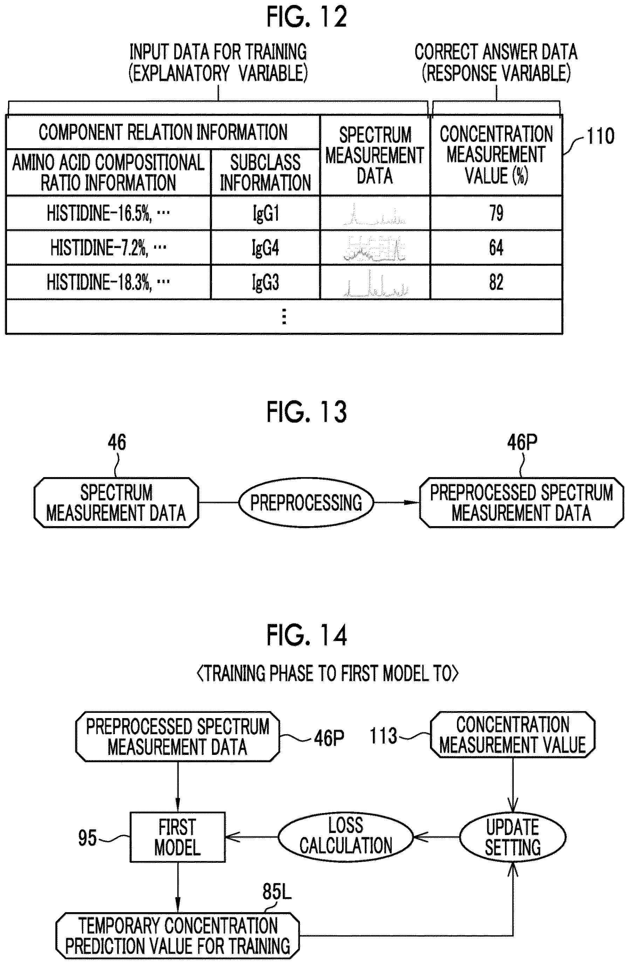

As shown in Fig. 12 as an example, the training data group 110 is stored in the storage 112 in a form of a data table. The spectrum measurement data 46 and the component relation information 47 in the training data 111 are used as input data for training (explanatory variables), and the concentration measurement value 113 is used as correct answer data (response variables). It should be noted that the training data 111 in which IgG2, which is the subclass of the target antibody 19T, is registered in the subclass information 56 does not exi st.

As shown in Fig. 13 as an example, the learning apparatus performs the same preprocessing as the preprocessing via the preprocessing unit 78 on the spectrum measurement data 46 of the training data 111, to obtain preprocessed spectrum measurement data 46P.

The sparse processing shown in Fig. 8 in the preprocessing is performed based on sparse modeling with respect to the spectrum measurement data 46 of the training data 111. The sparse modeling in the present embodiment means that the explanatory variables are sorted, that is, some of the explanatory variables are excluded for a regression model in which the intensity for each wave number included in the spectrum measurement data 46 of the training data 111 is included as the explanatory variable and the concentration prediction value 85 is included as the response variable. As the method of the sparse modeling, for example, a method using a least absolute shrinkage and selection operator (Lasso) regression can be used. The Lasso regression is a method of sorting the explanatory variable so that a loss function calculated by adding a penalty term (also referred to as a penalty term) to a root mean squared error (RMSE) is minimized. The penalty term is determined by, for example, cross-validation represented by K-fold cross-validation.

The sparse modeling is performed by the following procedure. First, the processing of thinning out the intensity at a randomly determined wave number is performed on the spectrum measurement data 46 of the training data 111, and a regression model indicating a relationship between the spectrum measurement data 46 after the thinning out processing and the corresponding concentration prediction value 85 is constructed. Then, the loss function in which the penalty term is added to the RMSE is derived for the constructed regression model. By repeating a predetermined number of times each processing of the thinning out, the construction of the regression model, and the derivation of the loss function, the regression model is generated for each of a plurality of spectrum measurement data 46 having different thinned out wave numbers, and a plurality of loss functions are derived for each regression model. Then, the intensity having the smallest number of cases in which the loss function can be minimized is sorted as the intensity having the relatively high correlation with the concentration prediction value 85, and the other intensities are excluded as the intensities having the relatively low correlation with the concentration prediction value 85.

As shown in Fig. 14 as an example, in a training phase of the first model 95, the learning apparatus inputs the preprocessed spectrum measurement data 46P of the training data 111 to the first model 95, and causes the first model 95 to output the temporary concentration prediction value for training 85TL. The temporary concentration prediction value for training 85TL is an example of a "state prediction result for training" according to the technology of the present disclosure.

The learning apparatus performs a loss calculation of the first model 95 using the loss function based on a result of comparison between the temporary concentration prediction value for training 85TL and the concentration measurement value 113. The learning apparatus performs the update setting of the coefficient between the nodes ND of the first model 95 in accordance with a result of the loss calculation, and updates the first model 95 in accordance with the update setting.

In the training phase of the first model 95, the learning apparatus repeatedly performs the series of processing of inputting the preprocessed spectrum measurement data 46P to the first model 95, causing the first model 95 to output the temporary concentration prediction value for training 85TL, performing the loss calculation, performing the update setting, and updating the first model 95, while changing the training data 111. The learning apparatus finishes the repetition of the series of processing in a case in which the prediction accuracy of the temporary concentration prediction value for training 85TL for the concentration measurement value 113 reaches a predetermined set level. The first model 95 in which the prediction accuracy reaches the set level is stored in the storage 60 as a part of the trained concentration predictive model 71, and is used by the prediction unit 79. It should be noted that the training may be finished in a case in which the series of processing are repeated a set number of times, regardless of the prediction accuracy of the temporary concentration prediction value for training 85TL for the concentration measurement value 113.

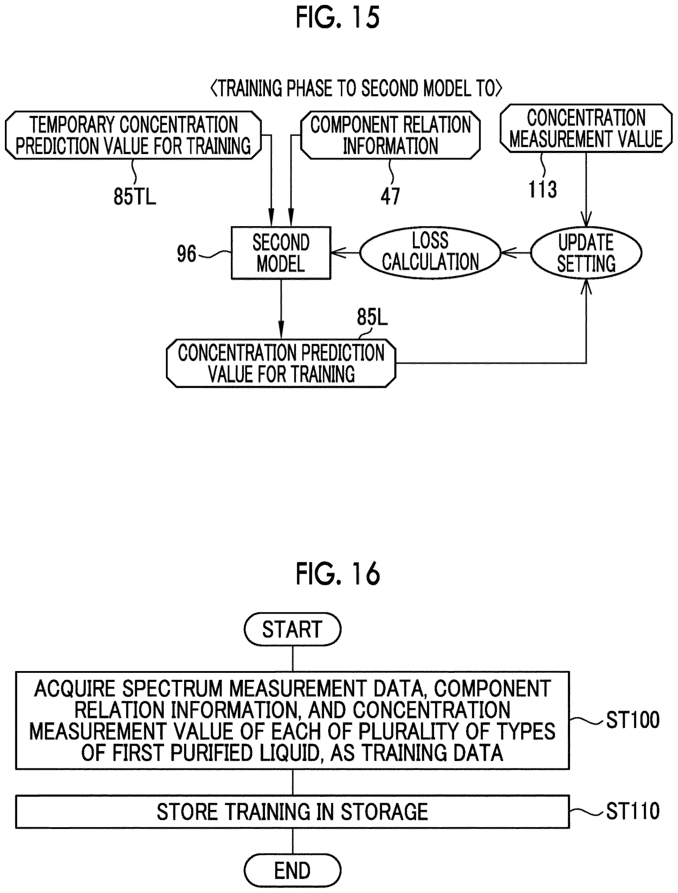

As shown in Fig. 15 as an example, in a training phase of the second model 96, the learning apparatus inputs the component relation information 47 in the training data 111 and the temporary concentration prediction value for training 85TL output by the first model 95 to the second model 96, and causes the second model 96 to output a concentration prediction value for training 85L. The concentration prediction value for training 85L is also an example of a "state prediction result for training" according to the technology of the present disclosure, similarly to the temporary concentration prediction value for training 85TL.

The learning apparatus performs a loss calculation of the second model 96 using the loss function based on a result of comparison between the concentration prediction value for training 85L and the concentration measurement value 113. The learning apparatus performs the update setting of the coefficient between the nodes ND of the second model 96 in accordance with a result of the loss calculation, and updates the second model 96 in accordance with the update setting.

In the training phase of the second model 96, the learning apparatus repeatedly performs the series of processing of inputting the component relation information 47 and the temporary concentration prediction value for training 85TL to the second model 96, causing the second model 96 to output the concentration prediction value for training 85L, performing the loss calculation, performing the update setting, and updating the second model 96, while changing the training data 111. The learning apparatus finishes the repetition of the series of processing in a case in which the prediction accuracy of the concentration prediction value for training 85L for the concentration measurement value 113 reaches a predetermined set level. The second model 96 in which the prediction accuracy reaches the set level is stored in the storage 60 as a part of the trained concentration predictive model 71, and is used by the prediction unit 79. It should be noted that the training may be finished in a case in which the series of processing are repeated a set number of times, regardless of the prediction accuracy of the concentration prediction value for training 85L for the concentration measurement value 113.

The learning apparatus performs the training of the second model 96 after the training of the first model 95 is finished. Of course, the training of the first model 95 and the training of the second model 96 may be performed in parallel.

Next, actions of the above-described configuration will be described with reference to the flowcharts shown in Figs. 16 to 19.

As shown in Fig. 16 as an example, first, a plurality of training data 111 for generating the trained concentration predictive model 71 are acquired by the learning apparatus (step ST100). More specifically, as shown in Fig. 11, at least two types of the training data 111 are acquired, such as the training data 111A acquired from the antibody 19A of which the subclass is IgG 1 and the first purified liquid 28A containing the antibody 19A, and the training data 111B acquired from the antibody 19B of which the subclass is IgG4 and the first purified liquid 28B containing the antibody 19B. The training data 111 is stored in the storage 112 of the learning apparatus (step ST110).

As shown in Fig. 13, the preprocessing is performed on the spectrum measurement data 46 of the training data 111, to obtain the preprocessed spectrum measurement data 46P.

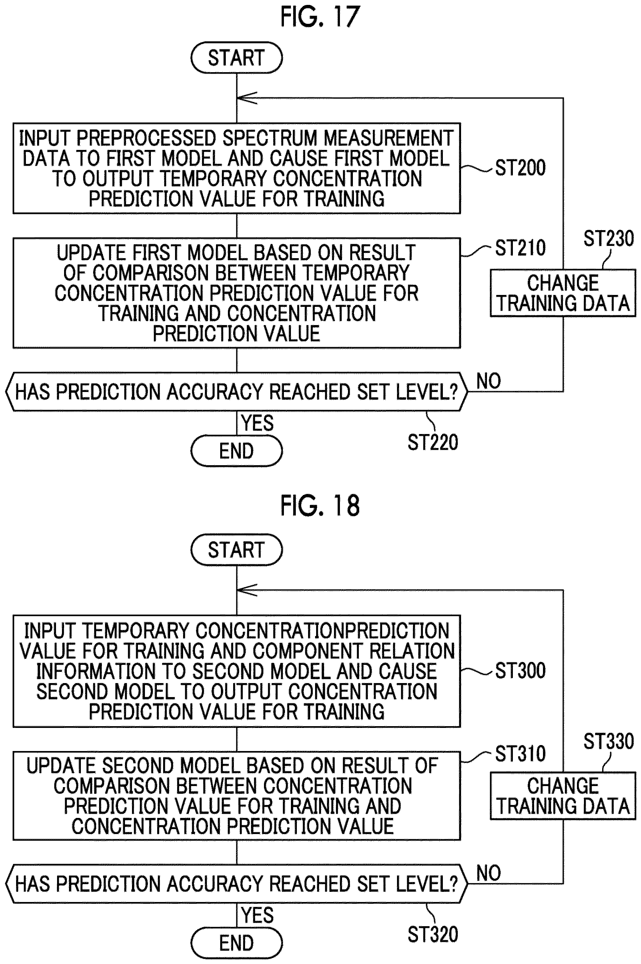

In the learning apparatus, the first model 95 shown in Fig. 14 is trained. As shown in Fig. 17 as an example, the preprocessed spectrum measurement data 46P is input to the first model 95, and thus the temporary concentration prediction value for training 85TL is output from the first model 95 (step ST200). Then, the first model 95 is updated based on the result of comparison between the temporary concentration prediction value for training 85TL and the concentration measurement value 113 (step ST210). The pieces of processing of step ST200 and step ST210 are repeated, in a period in which the prediction accuracy of the temporary concentration prediction value for training 85TL for the concentration measurement value 113 does not reach the set level (NO in step ST220), while changing the training data 111 (step ST230). In a case in which the prediction accuracy of the temporary concentration prediction value for training 85TL for the concentration measurement value 113 reaches the set level (YES in step ST220), the processing is finished.

In the learning apparatus, the second model 96 shown in Fig. 15 is trained. As shown in Fig. 18 as an example, the temporary concentration prediction value for training 85TL and the component relation information 47 are input to the second model 96, and thus the concentration prediction value for training 85L is output from the second model 96 (step ST300). Then, the second model 96 is updated based on the result of comparison between the concentration prediction value for training 85L and the concentration measurement value 113 (step ST310). The pieces of processing of step ST300 and step ST310 are repeated, in a period in which the prediction accuracy of the concentration prediction value for training 85L for the concentration measurement value 113 does not reach the set level (NO in step ST320), while changing the training data 111 (step ST330). In a case in which the prediction accuracy of the concentration prediction value for training 85L for the concentration measurement value 113 reaches the set level (YES in step ST320), the processing is finished. The first model 95 and the second model 96 trained in this way are collectively stored in the storage 60 of the information processing apparatus 45 as the trained concentration predictive model 71.

In a case in which the operation program 70 is activated in the information processing apparatus 45, the CPU 62 of the information processing apparatus 45 functions as the reception unit 75, the acceptance unit 76, the RW control unit 77, the preprocessing unit 78, the prediction unit 79, and the display control unit 80, as shown in Fig. 6.

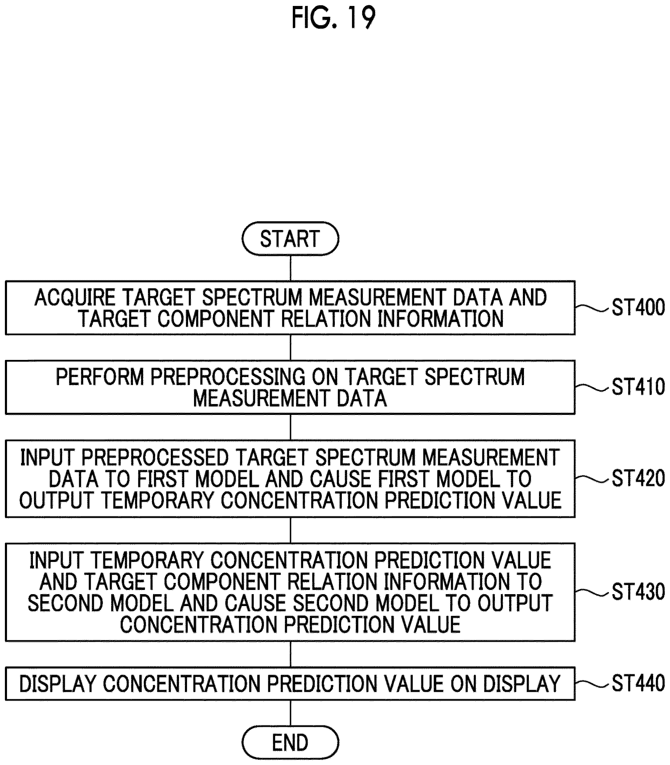

As an example, as shown in Fig. 19, the target spectrum measurement data 46T from the Raman spectrometer 40 is received by the reception unit 75. The acceptance unit 76 accepts the target component relation information 47T input by the operator via the input device 51. As a result, the target spectrum measurement data and the target component relation information 47T are acquired by the CPU 62 (step ST400). The target spectrum measurement data 46T is output from the reception unit 75 to the RW control unit 77, and is stored in the storage 60 by the RW control unit 77. The target component relation information 47T is output from the acceptance unit 76 to the RW control unit 77, and is stored in the storage 60 by the RW control unit 77.

The target spectrum measurement data 46T is read out from the storage 60 by the RW control unit 77, and is output to the preprocessing unit 78. In the preprocessing unit 78, as shown in Figs. 7 and 8, the preprocessing is performed on the target spectrum measurement data 46T, to obtain the preprocessed target spectrum measurement data 46TP (step ST410). The preprocessed target spectrum measurement data 46TP is output from the preprocessing unit 78 to the prediction unit 79.

The target component relation information 47T is read out from the storage 60 by the RW control unit 77, and is output to the prediction unit 79. In the prediction unit 79, as shown in Fig. 9, first, the preprocessed target spectrum measurement data 46TP is input to the first model 95, and thus the temporary concentration prediction value 85T is output from the first model 95 (step ST420). Subsequently, the temporary concentration prediction value 85T and the target component relation information 47T are input to the second model 96, and thus the concentration prediction value 85 is output from the second model 96 (step ST430). The concentration prediction value 85 is output from the prediction unit 79 to the display control unit 80.

Under the control of the display control unit 80, the notification screen of the concentration prediction value 85 is displayed on the display 50 (step ST440). As a result, the operator is notified of the concentration prediction value 85. The operator verifies whether or not the purification via the immunoaffinity chromatography device 25 is appropriately performed based on the concentration prediction value 85, or considers changing the purification conditions of the immunoaffinity chromatography device 25. The purification conditions are, for example, a flow rate in a case of injecting the culture supernatant liquid 17 into the column, and an amount and a composition of a buffer used in a case of eluting the antibody 19 from the column. It should be noted that the prediction unit 79 may output the concentration prediction value 85 to the RW control unit 77, and the RW control unit 77 may store the concentration prediction value 85 in the storage 60.

As described above, the CPU 62 of the information processing apparatus 45 uses the trained concentration predictive model 71 trained by using at least two types of the training data 111, such as the training data 111A and the training data 111B. The training data 111A includes the spectrum measurement data 46A obtained from the first purified liquid 28A containing the antibody 19A and the component relation information 47A related to the antibody 19A as the input data for training (explanatory variable), and includes the concentration measurement value 113A obtained by actually measuring the concentration of the antibody 19A in the first purified liquid 28A as the correct answer data (response variable). The training data 111B includes the spectrum measurement data 46B obtained from the first purified liquid 28B containing the antibody 19B and the component relation information 47B related to the antibody 19B as the input data for training (explanatory variable), and includes the concentration measurement value 113B obtained by actually measuring the concentration of the antibody 19B in the first purified liquid 28B as the correct answer data (response variable).

The CPU 62 acquires the target component relation information 47T related to the target antibody 19T, and the target spectrum measurement data 46T obtained from the target first purified liquid 28T containing the target antibody 19T. The CPU 62 applies the target component relation information 47T and the target spectrum measurement data 46T to the trained concentration predictive model 71, and causes the trained concentration predictive model 71 to output the concentration prediction value 85 of the target antibody 19T in the target first purified liquid 28T.

Since the component relation information 47 is also taken into account in addition to the spectrum measurement data 46, the concentration of the target antibody 19T of the subclass that does not exist in the training data 111 can also be predicted with high accuracy. Therefore, it is not necessary to generate a dedicated trained concentration predictive model 71 specialized for each antibody 19, and the prediction of the concentration of a plurality of types of the antibodies 19 can be performed by one trained concentration predictive model 71. Therefore, it is possible to efficiently predict the concentration of the target antibody 19T.

The trained concentration predictive model 71 comprises the first model 95 and the second model 96. The first model 95 outputs the temporary concentration prediction value 85T in accordance with the target spectrum measurement data 46T. The second model 96 outputs the concentration prediction value 85 in accordance with the target component relation information 47T and the temporary concentration prediction value 85T. In this way, the prediction accuracy of the concentration prediction value 85 can be increased by performing the prediction of the concentration of the target antibody 19T in two stages in a form of correcting the temporary concentration prediction value 85T predicted from the target spectrum measurement data 46T by using the target component relation information 47T.

The target antibody 19T is different from the antibody 19 (antibodies 19A and 19B, and the like) used to obtain the training data 111. Therefore, the effect that the concentration of the target antibody 19T can be efficiently predicted can be further exhibited. It should be noted that the concentration of the target antibody 19T having the same subclass as the antibody 19 of the training data 111 (in the present example, the target antibody 19T having the same subclass as the antibody 19A, that is, the target antibody 19T having the subclass of IgG1, or the target antibody 19T having the same subclass as the antibody 19B, that is, the target antibody 19T having the subclass of IgG4) can also be predicted.

The preprocessing unit 78 performs the preprocessing including the noise removal processing 90, the peak separation processing 91, and the peak emphasis processing 92 on the target spectrum measurement data 46T. The prediction unit 79 applies the preprocessed target spectrum measurement data 46TP to the trained concentration predictive model 71. Therefore, the prediction accuracy of the concentration prediction value 85 can be further increased.

It should be noted that the preprocessing unit 78 need only perform at least any one of the noise removal processing 90, the peak separation processing 91, or the peak emphasis processing 92, as the preprocessing. More specifically, the preprocessing unit 78 need only perform at least any one (or two or more combinations) of the smoothing processing, the baseline correction processing, the differentiation processing, the normalization processing, the averaging processing, the dimension reduction processing, or the sparse processing, as the preprocessing.

The bio-pharmaceutical containing the antibody 19 as the protein, which is called an antibody pharmaceutical, is widely used for the treatment of rare diseases such as hemophilia and Crohn's disease in addition to the treatment of chronic diseases such as cancer, diabetes, and rheumatoid arthritis. Therefore, according to the present example in which the first component, the second component, and the target component are the protein and the protein is the antibody 19, it is possible to promote the development of antibody pharmaceutical widely used for the treatment of various diseases.

It should be noted that the biological molecule and the target component are not limited to the protein. The biological molecule may be a peptide, a nucleic acid (DNA or ribonucleic acid (RNA)), a lipid, a virus, a virus subunit, a virus-like particle, or the like. The biological molecule may be a molecule synthesized by a chemical synthesis method.

The target component is not limited to the antibody 19. Impurities such as the cell-derived protein/cell-derived DNA 20 and the aggregate 21 may be used as the target component. In a case in which impurities other than the target protein are mixed in the bio-pharmaceutical, it is also important to predict the state of the impurities because the impurities may affect the pharmacological effect of the bio-pharmaceutical even in a case in which the amount thereof is trace.

The first suspension, the second suspension, and the target suspension are not limited to the first purified liquid 28 described as an example. The culture supernatant liquid 17, the second purified liquid 29, or the third purified liquid 30 may be used. The culture liquid in the culture tank 16 may be used.

The component relation information 47A, the component relation information 47B, and the target component relation information 47T include amino acid compositional ratio information 55A of the antibody 19A, amino acid compositional ratio information 55B of the antibody 19B, and the target amino acid compositional ratio information 55T of the target antibody 19T. The amino acid compositional ratio information 55A, the amino acid compositional ratio information 55B, and the target amino acid compositional ratio information 55T are information well representing the characteristics of the antibody 19A, the antibody 19B, and the target antibody 19T. The component relation information 47A, the component relation information 47B, and the target component relation information 47T include the subclass information 56A of the antibody 19A, the subclass information 56B of the antibody 19B, and the target subclass information 56T of the target antibody 19T. The subclass information 56A, the subclass information 56B, and the target subclass information 56T are also information well representing the characteristics of the antibody 19A, the antibody 19B, and the target antibody 19T. Therefore, the prediction accuracy of the concentration prediction value 85 can be further increased.

It should be noted that the target component relation information 47T may include isotype information of the target antibody 19T, such as IgA, IgD, IgE, IgG, and IgM, instead of or in addition to the target amino acid compositional ratio information 55T and the target subclass information 56T. In addition, the target component relation information 47T may include amino acid sequence information in which the order of the peptide bonds of the amino acids constituting the target antibody 19T is described from an amino terminal to a carboxyl terminal. The target component relation information 47T may further include a molecular weight, an isoelectric point, a molar absorption coefficient, and the like of the target antibody 19T. In a case in which the target component is DNA or RNA, information on a nucleic acid sequence of the DNA or the RNA can be used as the target component relation information 47T.

The Raman spectrum easily reflects information derived from a functional group of the amino acid in the protein. Therefore, by using the spectrum as the Raman spectrum as in the present example, the prediction accuracy of the concentration prediction value 85 of the target antibody 19T which is the protein can be further increased.

It should be noted that the spectrum is not limited to the Raman spectrum. An infrared absorption spectrum, a nuclear magnetic resonance spectrum, an ultraviolet-visible absorption spectroscopy (UV-Vis) spectrum, or a fluorescence spectrum may be used.

The trained concentration predictive model 71 is a machine learning model trained using the training data 111. The machine learning model is generally used for prediction of unknown parameters, and the prediction accuracy can be increased to a certain level by learning. Therefore, it is possible to easily generate the trained concentration predictive model 71 having a relatively high prediction accuracy.

It should be noted that the calibrated state predictive model is not limited to the trained concentration predictive model 71. A calibrated state predictive model generated by multivariate analysis or statistical analysis may be used. Examples of the multivariate analysis and the statistical analysis include multiple regression, partial least squares regression (PLS), principal component regression, logistic regression, Lasso regression, ridge regression, support vector regression, and Gaussian process regression. In the calibrated state predictive model generated by such a multivariate analysis and such a statistical analysis, a determination of coefficients of a regression equation based on at least two types of the calibration data corresponds to "calibration" according to the technology of the present disclosure.

Although the neural network 100 has been described as the trained concentration predictive model 71 (first model 95 and second model 96), the present disclosure is not limited to this. A decision tree, a random forest, a naive Bayes, a gradient boosting decision tree, or the like may be used.

The concentration is the most popular indicator for understanding the physicochemical features of the target antibody 19T. Therefore, in a case in which the concentration is predicted as the state of the target antibody 19T as in the present example, the operator can easily understand the physicochemical features of the target antibody 19T.

It should be noted that the state of the target antibody 19T is not limited to the concentration. A purity or a density of the target antibody 19T may be used. The purity is calculated by using the sum of the amount of the target antibodies 19T and the amount of impurities as a denominator, and using the amount of the target antibody 19T as a numerator. For example, two or more indicators such as the concentration and the density may be predicted. In addition, a quantitative indicator is not limited to the concentration and the purity, and a qualitative indicator such as a level of the quality of the target antibody 19T (two levels of good or bad, or five levels of 1 to 5, or the like) may be used.

In a case in which the target component is, for example, peptide, a compositional ratio of the amino acid in the peptide, a content compositional ratio of the produced mixture (for example, a content compositional ratio of glucose to glutamic acid), or the like may be adopted as the state of the target component. In a case in which the target component is, for example, a chemical substance such as alcohol produced from cells, a polymerization rate may be adopted as the state of the target component. In these cases as well, similarly to a case of the target antibody 19T, two or more indicators may be predicted, or the qualitative indicator may be predicted.

In the training phase of the first model 95 and the second model 96, the spectrum measurement data 46 and the component relation information 47 (explanatory variable of the calibration data) of the training data 111 are input to the first model 95 and the second model 96 as the input data for training, to cause the first model 95 and the second model 96 to output the temporary concentration prediction value for training 85TL and the concentration prediction value for training 85L. Then, the first model 95 and the second model 96 are updated based on the result of comparison between the temporary concentration prediction value for training 85TL, the concentration prediction value for training 85L, and the concentration measurement value 113 (response variable of the calibration data) which is the correct answer data of the training data 111. Then, the trained concentration predictive model 71 is generated by repeating inputting the spectrum measurement data 46 and the component relation information 47 to the first model 95 and the second model 96, causing the first model 95 and the second model 96 to output the temporary concentration prediction value for training 85TL and the concentration prediction value for training 85L, and updating the first model 95 and the second model 96, while changing the training data 111. Therefore, it is possible to easily generate the trained concentration predictive model 71 having a relatively high prediction accuracy.

Hereinafter, an example of the technology of the present disclosure will be described.

In the present example, protein A affinity chromatography was carried out to simulate the third process 12 in order to collect the Raman spectrum of the suspension containing the antibody at various concentrations. As the suspension to be introduced into the affinity chromatography, first, the culture supernatant liquid of CHO cells that produce an antibody mAbA of the subclass IgG 1 was used. A protein A column (manufactured by Cytiva, product name MabSelect SuRe) was connected to an affinity chromatography device (manufactured by Cytiva, product name AKTA pure 25) and used.

In a chromatographic elution step in the affinity chromatography device, an acidic eluent was not switched in a stepwise manner, but the gradient elution in which the acidic eluent was allowed to flow in a gradient manner while a concentration gradient was applied was performed. The gradient elution conditions were set in the following three patterns. That is, a column volume (CV) of the protein A column was set in a range of 5 CV, 10 CV, and 15 CV Then, the Raman spectrum of the first purified liquid purified by affinity chromatography was measured by using the Raman spectrometer. It should be noted that the Raman spectrum was measured continuously during the progress of the affinity chromatography. The concentration of the antibody in the first purified liquid was actually measured by offline analysis of the first purified liquid. The spectrum measurement data of the Raman spectrum measured during the progress of the affinity chromatography and the concentration measurement value in a case of measuring the Raman spectrum were stored in association with each other.

Secondly, the same test as the antibody mAbA was carried out by using the culture supernatant liquid of the CHO cells that produce an antibody mAbB of the subclass IgG4, and the spectrum measurement data and the concentration measurement value were acquired. The chromatographic elution step was carried out only under one condition of 10 CV

From the amino acid sequence information of each of the antibody mAbA and the antibody mAbB, the amino acid compositional ratio information of each of the antibody mAbA and the antibody mAbB was acquired. The acquired amino acid compositional ratio information was used as the component relation information, and was used as the training data together with the spectrum measurement data and the concentration measurement value, which were acquired earlier.

The spectrum measurement data of the antibody mAbA was used as the input data for training, and the concentration measurement value of the antibody mAbA was used as the correct answer data, to construct the first model. In addition, the component relation information of each of the antibody mAbA and the antibody mAbB, and the temporary concentration prediction value for training output from the first model were used as the input data for training, and the concentration measurement value of each of the antibody mAbA and the antibody mAbB was used as the correct answer data, to construct the second model. As described above, the trained concentration predictive model that outputs the concentration prediction value in a case in which the spectrum measurement data and the component relation information are applied was generated.

In order to verify the prediction accuracy of the generated trained concentration predictive model, the same test as the antibody mAbA or the like was carried out by using the culture supernatant liquid of CHO cells that produce an antibody mAbC different from the antibodies mAbA and mAbB of the subclass IgG2, and the spectrum measurement data and the concentration measurement value were acquired. The chromatographic elution step was carried out only under one condition of 10 CV Additionally, as the component relation information of the antibody mAbC, the amino acid compositional ratio information of the antibody mAbC was acquired from the amino acid sequence information of the antibody mAbC.

The spectrum measurement data and the component relation information of the antibody mAbC were applied to the trained concentration predictive model generated as described above, to cause the trained concentration predictive model to output the concentration prediction value. In a case in which the concentration prediction value and the concentration measurement value were compared to calculate a prediction error of the trained concentration predictive model, RMSE = 0.54. Therefore, it was confirmed that the concentration could be predicted with relatively high accuracy even for the antibody of a different type from the antibody used for the training data. It should be noted that the antibody mAbA and the antibody mAbB are examples of a "first component" and a "second component" according to the technology of the present disclosure. The antibody mAbC is an example of a "target component" according to the technology of the present disclosure.

The trained concentration predictive model may be generated for each isotype or each subclass of the antibody 19. In a case in which the trained concentration predictive model is generated for each subclass, the information on the light chain of the antibody 19, such as λ and κ, is used as the component relation information in addition to the subclass information 56.

In the above-described embodiment, the "types are different from each other" is defined by the different subclasses, but the "types are different from each other" may be defined by the different isotypes, or the "types are different from each other" may be defined by the different light chains.

The calibration data may be data related to one type of the antibody 19.

In the above-described embodiment, for example, as the hardware structure of the processing unit that executes various types of processing, such as the reception unit 75, the acceptance unit 76, the RW control unit 77, the preprocessing unit 78, the prediction unit 79, and the display control unit 80, various processors described below can be used. As described above, in addition to the CPU 62 which is a general-purpose processor that executes software (operation program 70) and that functions as various processing units, examples of the various processors include a programmable logic device (PLD) which is a processor of which a circuit configuration can be changed after manufacture, such as a field programmable gate array (FPGA), and a dedicated electric circuit which is a processor having a circuit configuration designed as a dedicated circuit in order to execute specific processing, such as an application specific integrated circuit (ASIC).

One processing unit may be configured by using one of these various processors, or may be configured by using a combination of two or more processors of the same type or different types (for example, a combination of a plurality of FPGAs and/or a combination of a CPU and an FPGA). Moreover, a plurality of processing units may be configured by one processor.

As an example in which the plurality of processing units are configured by using one processor, first, as represented by a computer such as a client and a server, there is a form in which one processor is configured by using a combination of one or more CPUs and software and this processor functions as the plurality of processing units. Second, as represented by a system on chip (SoC) or the like, there is a form in which a processor that realizes the functions of the entire system including the plurality of processing units with one integrated circuit (IC) chip is used. In this way, as the hardware structure, the various processing units are constituted by one or more of the various processors described above.

Further, more specifically, an electric circuit (circuitry) in which circuit elements such as semiconductor elements are combined can be used as the hardware structure of the various processors.

In the technology of the present disclosure, the above-described various embodiments and/or various modification examples may be combined with each other as appropriate. Further, it is needless to say that the present disclosure is not limited to the above-described embodiment and various configurations can be adopted without departing from the scope of the technology of the present disclosure. Furthermore, the technology of the present disclosure extends to a storage medium that non-temporarily stores a program, in addition to the program.

The above-described contents and above-shown contents are the detailed description of the parts according to the technology of the present disclosure, and are merely an example of the technology of the present disclosure. For example, the above description of the configurations, functions, actions, and effects is a description of an example of the configurations, functions, actions, and effects of the portions according to the technology of the present disclosure. Accordingly, it goes without saying that unnecessary portions may be deleted, new elements may be added, or replacement may be made with respect to the above-described contents and above-shown contents without departing from the scope of the technology of the present disclosure. In addition, in order to avoid complication and facilitate understanding of portions according to the technology of the present disclosure, description related to common technical knowledge or the like that does not need to be particularly described for enabling implementation of the technology of the present disclosure is omitted in the above-described contents and above-shown contents.

In the present specification, "A and/or B" has the same meaning as "at least one of A or B". That is, "A and/or B" means that only A may be used, only B may be used, or a combination of A and B may be used. In addition, in the present specification, in a case in which three or more matters are expressed by being connected by "and/or", the same concept as "A and/or B" is applied.

All documents, patent applications, and technical standards described in the present specification are incorporated in the present specification by reference to the same extent as a case in which each individual publication, patent application, or technical standard is specifically and individually indicated to be incorporated by reference.

## Claims

1. An information processing apparatus that predicts a state of a component in a suspension in which biological molecules are dispersed as the components in a liquid, based on spectrum measurement data obtained by measuring a spectrum of electromagnetic waves emitted from the suspension, the information processing apparatus comprising:
a processor,
wherein the processor
uses a calibrated state predictive model calibrated by using at least two types of calibration data of first calibration data and second calibration data,
the first calibration data including first spectrum measurement data, which is the spectrum measurement data obtained from a first suspension containing a first component, and first component relation information related to the first component as explanatory variables, and including first state relation information related to a state of the first component as a response variable, and
the second calibration data including second spectrum measurement data, which is the spectrum measurement data obtained from a second suspension containing a second component, and second component relation information related to the second component as explanatory variables, and including second state relation information related to a state of the second component as a response variable,
acquires target component relation information related to a target component, which is a target of which the state is predicted, and target spectrum measurement data, which is the spectrum measurement data obtained from a target suspension containing the target component, and
applies the target component relation information and the target spectrum measurement data to the calibrated state predictive model, and causes the calibrated state predictive model to output a target state prediction result obtained by predicting the state of the target component in the target suspension.

2. The information processing apparatus according to claim 1,
wherein the calibrated state predictive model includes
a first model that outputs a temporary prediction result of the state of the target component in accordance with the target spectrum measurement data, and
a second model that outputs the target state prediction result in accordance with the target component relation information and the temporary prediction result.

3. The information processing apparatus according to claim 1 or 2,
wherein the target component is different from a component used to obtain the calibration data.

4. The information processing apparatus according to any one of claims 1 to 3,
wherein the processor
performs preprocessing for at least any one of noise removal, peak separation, or peak emphasis on the target spectrum measurement data, and then applies the preprocessed target spectrum measurement data to the calibrated state predictive model.

5. The information processing apparatus according to any one of claims 1 to 4,
wherein the first component, the second component, and the target component are proteins.

6. The information processing apparatus according to claim 5,
wherein the first component relation information, the second component relation information, and the target component relation information include information on a compositional ratio of an amino acid in the protein.

7. The information processing apparatus according to claim 5 or 6,
wherein the protein is an antibody.

8. The information processing apparatus according to claim 7,
wherein the first component relation information, the second component relation information, and the target component relation information include information on a subclass of the antibody.

9. The information processing apparatus according to any one of claims 1 to 8,
wherein the spectrum is a Raman spectrum.

10. The information processing apparatus according to any one of claims 1 to 9,
wherein the calibrated state predictive model is a machine learning model trained using the calibration data as training data.

11. The information processing apparatus according to any one of claims 1 to 10,
wherein the state is a concentration,
the first state relation information is a measurement value of a concentration of the first component,
the second state relation information is a measurement value of a concentration of the second component, and
the target state prediction result is a prediction value of a concentration of the target component.

12. An operation method of an information processing apparatus that predicts a state of a component in a suspension in which biological molecules are dispersed as the components in a liquid, based on spectrum measurement data obtained by measuring a spectrum of electromagnetic waves emitted from the suspension, the operation method comprising:
using a calibrated state predictive model calibrated by using at least two types of calibration data of first calibration data and second calibration data,
the first calibration data including first spectrum measurement data, which is the spectrum measurement data obtained from a first suspension containing a first component, and first component relation information related to the first component as explanatory variables, and including first state relation information related to a state of the first component as a response variable, and
the second calibration data including second spectrum measurement data, which is the spectrum measurement data obtained from a second suspension containing a second component, and second component relation information related to the second component as explanatory variables, and including second state relation information related to a state of the second component as a response variable;
acquiring target component relation information related to a target component, which is a target of which the state is predicted, and target spectrum measurement data, which is the spectrum measurement data obtained from a target suspension containing the target component; and
applying the target component relation information and the target spectrum measurement data to the calibrated state predictive model, and causing the calibrated state predictive model to output a target state prediction result obtained by predicting the state of the target component in the target suspension.

13. An operation program of an information processing apparatus that predicts a state of a component in a suspension in which biological molecules are dispersed as the components in a liquid, based on spectrum measurement data obtained by measuring a spectrum of electromagnetic waves emitted from the suspension, the operation program causing a computer to execute a process comprising:
using a calibrated state predictive model calibrated by using at least two types of calibration data of first calibration data and second calibration data,
the first calibration data including first spectrum measurement data, which is the spectrum measurement data obtained from a first suspension containing a first component, and first component relation information related to the first component as explanatory variables, and including first state relation information related to a state of the first component as a response variable, and
the second calibration data including second spectrum measurement data, which is the spectrum measurement data obtained from a second suspension containing a second component, and second component relation information related to the second component as explanatory variables, and including second state relation information related to a state of the second component as a response variable;
acquiring target component relation information related to a target component, which is a target of which the state is predicted, and target spectrum measurement data, which is the spectrum measurement data obtained from a target suspension containing the target component; and
applying the target component relation information and the target spectrum measurement data to the calibrated state predictive model, and causing the calibrated state predictive model to output a target state prediction result obtained by predicting the state of the target component in the target suspension.

14. A generation method of a calibrated state predictive model that predicts a state of a component in a suspension in which biological molecules are dispersed as the components in a liquid, based on spectrum measurement data obtained by measuring a spectrum of electromagnetic waves emitted from the suspension, the generation method comprising:
acquiring at least two types of calibration data of first calibration data and second calibration data,
the first calibration data including first spectrum measurement data, which is the spectrum measurement data obtained from a first suspension containing a first component, and first component relation information related to the first component as explanatory variables, and including first state relation information related to a state of the first component as a response variable, and
the second calibration data including second spectrum measurement data, which is the spectrum measurement data obtained from a second suspension containing a second component, and second component relation information related to the second component as explanatory variables, and including second state relation information related to a state of the second component as a response variable; and
generating the calibrated state predictive model by using the calibration data.

15. The generation method of a calibrated state predictive model according to claim 14, further comprising:
inputting the explanatory variables of the calibration data to a machine learning model as input data for training, and causing the machine learning model to output a state prediction result for training obtained by predicting the state; and
updating the machine learning model based on a result of comparison between the state prediction result for training and the response variable of the calibration data,
wherein the machine learning model is made to be the calibrated state predictive model by repeatedly performing inputting the explanatory variables to the machine learning model, causing the machine learning model to output the state prediction result for training, and updating the machine learning model, while changing the calibration data.

16. A calibrated state predictive model that predicts a state of a component in a suspension in which biological molecules are dispersed as the components in a liquid, based on spectrum measurement data obtained by measuring a spectrum of electromagnetic waves emitted from the suspension,
wherein the calibrated state predictive model
is generated by using at least two types of calibration data of first calibration data and second calibration data,
the first calibration data including first spectrum measurement data, which is the spectrum measurement data obtained from a first suspension containing a first component, and first component relation information related to the first component as explanatory variables, and including first state relation information related to a state of the first component as a response variable, and
the second calibration data including second spectrum measurement data, which is the spectrum measurement data obtained from a second suspension containing a second component, and second component relation information related to the second component as explanatory variables, and including second state relation information related to a state of the second component as a response variable, and
causes a computer to execute a function of, in a case in which target component relation information related to a target component, which is a target of which the state is predicted, and target spectrum measurement data, which is the spectrum measurement data obtained from a target suspension containing the target component, are applied, outputting a target state prediction result obtained by predicting the state of the target component in the target suspension.

17. An information processing apparatus that stores the calibrated state predictive model according to claim 16.
